# FASCICULE DE BREVET EUROPEEN

(11) **EP 3 749 399 B1**
(45) Date de publication et mention de la délivrance du brevet: **05.06.2024**
(21) Numéro de dépôt: 19705135.2
(22) Date de dépôt: 05.02.2019
(51) Int. Cl.: A61M 15/00, A61M 11/00, A61M 15/08

(54) **DISPOSITIF D'ASSISTANCE À L'UTILISATION D'UN DISPOSITIF DE DISTRIBUTION À ACTIVATION AXIALE**
VORRICHTUNG ZUR UNTERSTÜTZUNG BEI DER VERWENDUNG EINER AXIAL AKTIVIERTEN AUSGABEVORRICHTUNG
DEVICE FOR ASSISTING IN THE USE OF AN AXIALLY ACTIVATED DISPENSING DEVICE

(30) Priorité: 06.02.2018 FR 1850991
(43) Date de publication de la demande: 16.12.2020
(73) Titulaire: NEMERA LA VERPILLIERE, 38290 La Verpillière (FR)
(72) Inventeur: DECOCK, Thierry, 69007 Lyon (FR); CASSAGNE, Loïck, 38080 Saint-Alban-de-Roche (FR); PINTUS, Pierre, 38300 Crachier (FR)
(74) Mandataire: LLR
(86) Numéro de dépôt international: PCT/EP2019/052778
(87) Numéro de publication internationale: WO 2019/154808

(56) Documents cités:
- WO-A1-2017/178865
- WO-A1-2017/205824
- US-A1- 2011 041 845
- US-A1- 2012 055 472
- US-A1- 2012 080 029

## Description

L'invention concerne un dispositif d'assistance à l'utilisation d'un dispositif de distribution à activation axiale et un procédé de surveillance de la distribution d'une dose complète lors d'une activation d'un dispositif de distribution de produit.

Quand on distribue un produit, il est souvent intéressant de connaître la quantité de produit distribuée. Cela est particulièrement vrai pour l'administration de produits médicaux, pour laquelle la quantité de médicament administrée doit être contrôlée précisément selon la prescription. Une prise insuffisante ou une surdose de médicaments est à éviter pour préserver la santé du patient, ou encore cela permet par la suite de savoir la quantité restante de produit dans le dispositif de distribution.

Le document WO 2017/178767 décrit un dispositif de distribution de produit par inhalation. Le dispositif de distribution est notamment capable de détecter les déplacements du réservoir contenant le produit au moyen d'un capteur de contact. En effet, quand le réservoir se déplace pour distribuer une dose, il entre en contact avec le capteur de contact qui est disposé sur sa trajectoire. Cela permet de déduire l'administration d'une dose et de compter le nombre de doses distribuées. Cependant, ce dispositif ne donne pas d'information en lien avec la quantité d'une dose distribuée, en particulier si la dose distribuée a été complète ou incomplète.
Le document WO 2017/205824 divulgue un dispositif d'assistance permettant de guider l'utilisation d'un dispositif de distribution de produit de type inhalateur ou nasal lors de la préparation et de la distribution du médicament.
Le document WO 2017/178865 décrit un dispositif additionnel à un inhalateur-doseur, lequel permet d'être manipulé de manière simple lorsqu'il est fixé sur l'inhalateur, tout en permettant l'acquisition et le suivi de données relatives à l'observance et la conformité de la distribution d'un médicament.

L'invention a notamment pour but de fournir un dispositif permettant de détecter une activation distribuant une dose complète de produit.

À cet effet, l'invention a pour objet un dispositif d'assistance à l'utilisation d'un dispositif de distribution à activation axiale d'un produit contenu dans un réservoir, tel que défini dans la revendication 1.

Ainsi, on propose de quantifier une valeur physique liée à la sollicitation exercée sur la zone d'appui entre la position de repos et la position activée. Ceci permet de déterminer par la suite si une dose a ou non été distribuée, et de façon encore plus avantageuse, si la dose distribuée est ou non complète, *i.e.* si la quantité de produit distribuée correspond à une quantité fixe prédéterminée de produit ou à une partie seulement de cette quantité. En effet, certaines doses peuvent varier selon différents paramètres physiques liés aux conditions d'activation du dispositif de distribution. Une mauvaise utilisation du dispositif peut provoquer l'administration d'une dose incomplète, c'est-à-dire une dose quantitativement inférieure à la quantité prédéterminée. Or, si cette dose incomplète est considérée comme complète, il peut y avoir un mauvais suivi de la posologie.

Par dose « incomplète » administrée, on comprend une dose expulsée de façon incomplète du dispositif de distribution, ou encore une dose expulsée entièrement, mais de façon incorrecte, c'est-à-dire mal délivrée, en raison d'une mauvaise utilisation du dispositif de distribution pouvant par exemple provoquer une expulsion trop lente ou inefficace du produit liquide, avec une mauvaise répartition spatiale des gouttes d'un spray ou un profil de spray ou pulvérisation non adapté.

De plus, le fait de quantifier la sollicitation de l'utilisateur exercée sur la zone d'appui permet par la suite de connaître le nombre résiduel de doses dans le réservoir. On peut en outre éviter que ce nombre soit faussé par la distribution d'une dose incomplète considérée comme complète. Cette information est susceptible d'intéresser l'utilisateur, lui permettant de connaître la situation dans le réservoir, généralement inconnue depuis l'extérieur. L'information peut également être utilisée pour d'autres types d'analyses ou de traitements visant à obtenir des informations supplémentaires.

On notera que l'« information sur la dose de produit distribuée » peut prendre de multiples formes. Elle peut être une information qualitative, de type dose « non délivrée » ou « dose délivrée », et/ou elle peut être en outre, quand la dose est délivrée, une information quantitative sur cette dose. Par ailleurs, que l'information sur la dose de produit distribuée soit qualitative et/ou quantitative, le système de traitement peut avantageusement fournir une information quantitative à partir de la valeur physique quantifiée en la combinant, ou non, avec d'autres informations telles que les propriétés physiques ou chimiques du produit, la géométrie de l'orifice de distribution, des tableaux de valeurs prédéfinies. Cette information quantitative comprend par exemple la quantité exacte de la dose, par exemple un volume de dose, une taille de goutte ou encore une information quantitative relative à la répartition spatiale d'un spray ou d'une goutte.

On comprend qu'un « dispositif de distribution à activation axiale » comprend tout type de dispositif de distribution dont la distribution de produit nécessite l'application d'une force par l'utilisateur dans l'axe de distribution du produit. Le dispositif de distribution peut être un dispositif de distribution d'un produit sous diverses formes, tout particulièrement sous forme de spray ou sous forme de gouttes, ou encore un dispositif de distribution d'un aérosol. Par ailleurs, le dispositif de distribution peut être un dispositif nasal, buccal, auriculaire, ophtalmique ou encore dermal.

La zone d'appui correspond à une zone montée mobile par rapport au réservoir entre la position de repos et la position activée. De préférence, l'utilisateur appuie directement sur la zone d'appui, néanmoins il peut également appuyer indirectement dessus. Par ailleurs la zone d'appui peut être portée par le dispositif d'assistance et/ou le dispositif de distribution. Le cas dans lequel la zone d'appui est portée par le dispositif d'assistance est avantageux en ce que cette zone d'appui du dispositif d'assistance peut en outre augmenter la surface de préhension par l'utilisateur par rapport à celle du dispositif de distribution seul, ce qui est particulièrement avantageux notamment pour des utilisateurs atteints de maladies neuromusculaires.

On comprend par ailleurs que les « moyens pour quantifier une valeur physique liée à la sollicitation exercée par l'utilisateur sur la zone d'appui entre la position de repos et la position activée » sont configurés pour être disposés directement ou indirectement sur la zone d'appui du dispositif d'assistance. Ils peuvent être disposés au voisinage de cette zone d'appui, ou à une certaine distance, comme cela peut être le cas lorsque ces moyens comprennent un capteur de pression disposé en-dessous du réservoir.

Le dispositif d'assistance peut en outre comporter l'une ou plusieurs des caractéristiques suivantes, prises seules ou en combinaison.
- Les moyens pour quantifier une valeur physique comprennent des moyens pour mesurer la distance de déplacement de la zone d'appui entre la position de repos et la position activée. En effet, le déplacement de la zone d'appui entre les deux positions est significatif de la distribution d'une dose. En mesurant la distance de ce déplacement et en fournissant cette information au système de traitement, celui-ci peut déterminer si la dose distribuée a été distribuée et si elle est complète ou non, par exemple en comparant la distance mesurée à un seuil de distance prédéterminé. On comprend que la distance de déplacement de la zone d'appui entre ses positions de repos et activée est une distance de déplacement relative par rapport au réservoir et qui correspond à la distance de déplacement du réservoir par rapport à la zone d'appui lors de l'activation du dispositif de distribution. Ainsi, on entend par moyens pour mesurer la distance de déplacement de la zone d'appui entre la position de repos et la position activée des moyens qui mesurent directement la distance de déplacement de la zone d'appui, ou des moyens qui mesurent indirectement la distance de déplacement de la zone d'appui en mesurant la distance de déplacement du réservoir par rapport à la zone d'appui.
- Les moyens pour quantifier une valeur physique comprennent des moyens pour mesurer la durée de déplacement de la zone d'appui par rapport au réservoir, le système de traitement des informations étant apte à fournir une information sur la vitesse de déplacement de la zone d'appui par rapport au réservoir. On propose ainsi d'obtenir la vitesse de déplacement de la zone d'appui entre ses deux positions, car c'est un paramètre qui influence la quantité de la dose distribuée. Il en ressort que le système de traitement des informations peut en déduire avantageusement une information quantitative sur la dose distribuée, par exemple en comparant la vitesse déduite à des courbes/abaques de vitesse prédéterminées. Comme pour les moyens pour mesurer la distance de déplacement de la zone d'appui, les moyens pour mesurer la durée de déplacement de la zone d'appui peuvent mesurer cette durée directement ou indirectement par le biais de la durée de déplacement du réservoir par rapport à la zone d'appui.
- Les moyens pour mesurer la distance et/ou la durée du déplacement de la zone d'appui comprennent des moyens optiques configurés pour mesurer la distance de déplacement, et de préférence la durée de déplacement, par l'émission et la réception d'un signal optique entre la zone d'appui et le réservoir. Les moyens optiques peuvent prendre diverses configurations. Le récepteur et l'émetteur peuvent être tous les deux disposés sur la zone d'appui (respectivement sur un élément fixe par rapport au réservoir, par exemple sur les moyens de solidarisation), le signal optique se réfléchit alors sur le réservoir (respectivement la zone d'appui) et l'on déduit la distance et/ou la durée de déplacement de la zone d'appui en mesurant la durée entre l'émission et la réception du signal optique, la vitesse du signal optique étant connue. Alternativement, le récepteur est disposé sur la zone d'appui (respectivement sur un élément fixe par rapport au réservoir) et l'émetteur sur un élément fixe par rapport au réservoir (respectivement la zone d'appui). Dans cette configuration, en comparant la durée entre l'émission et la réception du signal optique à la durée de référence sans déplacement de la zone d'appui, on obtient la distance et la vitesse de déplacement de la zone d'appui. On entend par « signal optique » toute onde électromagnétique, appartenant au spectre visible ou non. Les moyens optiques sont généralement des moyens capables d'émettre, recevoir et/ou réfléchir une telle onde.
- Les moyens pour quantifier une valeur physique comprennent des moyens pour mesurer la pression exercée sur la zone d'appui, pour fournir une information sur l'intensité et/ou la durée de la pression exercée par l'utilisateur sur la zone d'appui. Une pression d'intensité et/ou d'une durée suffisante exercée sur la zone d'appui dans la direction de distribution permet de déplacer la zone d'appui de sa position de repos à sa position activée et déclenche de ce fait la distribution d'une dose. Ainsi pour savoir si la dose distribuée est complète, on mesure l'intensité et/ou la durée de la pression exercée car ce sont des paramètres qui influencent la quantité de produit distribué. On comprend que les moyens pour mesurer la pression exercée sur la zone d'appui peuvent être montés fixes par rapport au réservoir. En effet, lorsque l'utilisateur exerce une pression sur la zone d'appui, cette pression est transmise au moins partiellement au réservoir, de sorte que des moyens de mesure de la pression solidaires du réservoir reçoivent une variation de pression significative d'une activation du dispositif de distribution.
- Les moyens pour quantifier une valeur physique comprennent des moyens pour mesurer l'accélération de la zone d'appui. Avantageusement, les moyens pour mesurer l'accélération comprennent un accéléromètre disposé directement sur la zone d'appui ou sur un élément monté fixe par rapport à la zone d'appui. Comme pour les moyens pour mesurer la distance et/ou la durée de déplacement de la zone d'appui les moyens pour mesurer l'accélération de la zone d'appui peuvent mesurer cette accélération directement ou indirectement par le biais de la durée de déplacement du réservoir par rapport à la zone d'appui. Dans ce second cas, l'accéléromètre peut être disposé soit directement sur le réservoir ou sur un élément monté fixe par rapport au réservoir, par exemple sur un corps principal logeant le réservoir. La combinaison de la mesure de la pression par les moyens décrits ci-dessus et de la mesure de l'accélération dans le temps est particulièrement avantageuse en ce qu'elle permet de déterminer si la dose distribuée est complète, tout en évitant les faux positifs. En effet, la mesure d'un accéléromètre seul ne permet pas forcément de savoir si la mesure obtenue est due à une accélération relative entre la zone d'appui et le réservoir ou à une accélération du dispositif de distribution pris dans son ensemble. Il est donc intéressant de la combiner avec les moyens de mesure de pression pour s'assurer que l'accélération mesurée est relative entre la zone d'appui et le réservoir. Par ailleurs, on constate que l'on peut avoir une dose incomplète distribuée si l'on appuie selon une intensité satisfaisante mais sur une durée trop courte, ou encore si l'on appuie assez longtemps mais pas assez fort. Grâce à la mesure combinée des deux paramètres, on peut déduire si une dose complète est délivrée et par la suite déduire le nombre restant de doses dans le dispositif de distribution.
- Un exemple d'élément fixe par rapport à la zone d'appui est un embout de distribution porté par le dispositif de distribution solidarisé au dispositif d'assistance. On comprend que l'embout de distribution est une partie du dispositif de distribution portant l'orifice de distribution et qui est mobile par rapport au réservoir du dispositif de distribution, son déplacement permettant d'activer la distribution de produit lors d'une pression exercée par l'utilisateur sur la zone d'appui. On notera que les moyens pour mesurer l'accélération peuvent comprendre un gyroscope électronique à la place de l'accéléromètre.
- Les moyens pour quantifier une valeur physique comprennent des moyens pour mesurer l'accélération relative entre la zone d'appui et le réservoir, de préférence deux accéléromètres, un premier accéléromètre étant monté fixe par rapport à la zone d'appui et un deuxième accéléromètre étant monté fixe par rapport au réservoir, le système de traitement des informations étant apte à traiter les informations d'accélération des deux accéléromètres pour en déduire l'accélération relative entre la zone d'appui et le réservoir. La présence des deux accéléromètres permet de mesurer l'accélération relative de la zone d'appui par rapport au réservoir, et éviter ainsi des faux positifs. En effet par exemple, lorsque la zone d'appui et le réservoir se déplacent de manière synchrone mais sont immobiles l'un par rapport à l'autre, les deux accéléromètres fournissent les mêmes mesures, donc on peut identifier un faux positif (ie. le premier accéléromètre indique un déplacement mais le second accéléromètre indique le même déplacement, ce déplacement n'est donc pas significatif d'une activation), sans avoir besoin de moyens pour mesurer la pression tels que décrits précédemment. La mesure de l'accélération relative entre la zone d'appui du dispositif d'assistance et le réservoir du dispositif de distribution permet de déduire la vitesse de la zone d'appui par rapport au réservoir au moment de son activation et ainsi fournir une information permettant de déduire la quantité de la dose distribuée.
- Le dispositif d'assistance comprend des moyens pour mesurer le poids du dispositif de distribution solidarisé au dispositif d'assistance, configurés pour fournir une information sur la quantité de produit dans le réservoir. La mesure du poids permet de donner une information directe sur la quantité de produit restante, la densité du produit étant a priori connue, par exemple en étant pré-enregistrée ou lue sur le dispositif de distribution. En outre en croisant la mesure du poids avec les informations fournies par les moyens pour quantifier une valeur physique, on a une information quantitative précise sur la dose distribuée et le nombre restant de doses. Cela permet par ailleurs de vérifier la délivrance correcte d'une dose. En particulier, si l'on ne détecte pas de changement de poids, on ne décrémente pas le nombre de doses. Avantageusement, les moyens pour mesurer le poids comprennent un capteur de poids, par exemple de type capteur de pression (« Force Sensing Resistor » ou FSR en anglais), en-dessous du réservoir et/ou au-dessus de ce dernier. Un exemple de capteur de poids convenable est le capteur de pression de la marque Interlink Electronics agissant dans la gamme de forces 20 gf - 2,0 kgf.
- Le système de traitement est configuré pour fournir une information quantitative sur la dose de produit distribuée, par exemple un volume de dose, une taille de goutte ou encore une information quantitative relative à la répartition spatiale d'un spray ou de gouttes. Pour obtenir cette information, le système de traitement utilise les informations sur la distance et/ou la vitesse de déplacement de la zone d'appui entre la position de repos et la position activée, et/ou l'accélération relative entre la zone d'appui et le réservoir, et/ou le poids du dispositif de distribution solidarisé au dispositif d'assistance.
- Le système de traitement comprend des moyens de lecture d'informations portées par le dispositif de distribution. Le système de traitement a alors accès à diverses informations sur le produit contenu dans le dispositif de distribution ou sur le dispositif de distribution lui-même, ne nécessitant pas un paramétrage manuel du dispositif d'assistance. Ces informations sont par exemple le nombre de doses théoriques contenues dans le réservoir, la quantité théorique d'une dose, les propriétés physiques ou chimiques du produit telles que la viscosité du produit, les dimensions de l'embout de distribution, des tableaux de valeurs prédéterminées, des outils de calculs. Une partie de ces informations peuvent être utiles au traitement des informations fournies par les différents moyens, par exemple les moyens pour quantifier une valeur physique. Le dispositif de distribution peut porter des informations sous forme de puces numériques, de bandes magnétiques, de codes barres ou tout autre type de support dont l'information portée est lisible par voie électronique.
- Le système de traitement comprend des moyens de stockage d'une valeur variable sur la quantité de produit restant dans le réservoir. La valeur variable est par exemple utilisée pour déduire la quantité de produit distribué et maîtriser la quantité de produit restant dans le réservoir. Elle est mise à jour lors d'une distribution de produit. En stockant ces valeurs, on peut suivre l'évolution du nombre de doses délivrées et/ou restantes dans le temps. Avantageusement, les moyens de stockage sont capables de stocker d'autres valeurs et informations utiles au système de traitement et/ou à l'utilisateur.
- Le dispositif d'assistance comprend des moyens d'indication des informations fournies par le système de traitement, par exemple des moyens visuels, des moyens sonores et/ou des moyens tactiles. Ces moyens permettent d'indiquer à l'utilisateur des informations sur la délivrance effective d'une dose, la distribution d'une dose complète ou incomplète, la quantité de produit délivrée et/ou restante et/ou d'autres informations sur le produit (par exemple lues par les moyens de lecture). Les moyens d'affichage peuvent comprendre un écran d'affichage d'informations sous forme alphanumérique et/ou des signaux lumineux, par exemple de couleur ou de forme différentes.
- Les moyens de solidarisation sont configurés pour être solidarisés à un dispositif de distribution comprenant une pompe. Un dispositif de distribution à pompe correspondant généralement à un dispositif sans aérosol. Il est particulièrement avantageux de pouvoir mesurer la pression exercée par l'utilisateur sur la zone d'appui et/ou la distance de déplacement et/ou la vitesse de déplacement de la zone d'appui car elle peut avoir un impact plus important sur la dose délivrée que dans le cas d'un aérosol. En effet, pour une pompe, seule l'action de l'utilisateur est moteur de la distribution, il n'y a pas de produit sous pression qui assiste la délivrance.
- Le dispositif d'assistance est configuré pour être solidarisé à un dispositif de distribution comprenant une valve de distribution d'un aérosol.
- Le dispositif d'assistance comprend des moyens pour activer et/ou réveiller les moyens pour quantifier une valeur physique, comprenant par exemple des moyens pour détecter la pression exercée sur la zone d'appui. Comme l'utilisateur doit exercer une pression pour déplacer la zone d'appui et distribuer une dose de produit, on peut décider de réveiller des composants à partir d'une certaine pression, qui peut être une pression minimale pour distribuer une dose de produit ou bien une pression inférieure à cette pression minimale. Ainsi, quantifier la ou les valeurs physiques à partir de ce moment permet de détecter chaque activation. Lorsqu'une telle pression n'est pas détectée, il est avantageux de faire travailler moins de composants pour économiser l'énergie et simplifier les données. Par « réveiller des composants » on comprend allumer des composants électroniques ou les faire sortir d'un mode d'économie d'énergie.
- La zone d'appui comprend deux surfaces d'appui disposées de part et d'autre d'un orifice de distribution qui est porté par le dispositif de distribution solidarisé au dispositif d'assistance, par exemple une collerette d'appui configurée pour recevoir le doigt index et le doigt majeur d'un utilisateur. La zone d'appui peut-être venue de matière avec un embout de distribution dans lequel est ménagé l'orifice de distribution, ou bien rapportée sur cet embout de distribution, notamment dans le cas où la zone d'appui est portée par le dispositif d'assistance.

L'invention concerne en outre un procédé de surveillance de la distribution d'une dose complète lors d'une activation d'un dispositif de distribution de produit, au moyen d'un dispositif d'assistance tel que décrit précédemment, comprenant les étapes suivantes :
- quantification d'une valeur physique liée à la sollicitation exercée par l'utilisateur sur la zone d'appui entre la position de repos et la position activée,
- qualification de la dose distribuée à partir de la valeur physique quantifiée.

En effet, la sollicitation exercée sur la zone d'appui entre la position de repos et la position activée, généré par l'application d'une force par l'utilisateur sur la zone d'appui, est significative de la distribution d'une dose. La quantification d'une valeur physique liée à cette sollicitation permet d'obtenir d'une part une information sur la distribution effective ou non d'une dose et d'autre part une information qualitative (complète ou incomplète) et/ou quantitative sur la dose distribuée (telle que quantité distribuée, répartition spatiale des gouttes, taille des gouttes...).

Le procédé de surveillance peut en outre comporter l'une ou plusieurs des caractéristiques suivantes, prises seules ou en combinaison.
- L'étape de quantification d'une valeur physique comprend une étape de mesure de la distance de déplacement de la zone d'appui entre la position de repos et la position activée. La distance de déplacement mesurée peut donner au moins une information qualitative sur la dose distribuée, à savoir si la dose distribuée est complète ou non.
- L'étape de quantification d'une valeur physique comprend une étape de mesure de la durée de déplacement de la zone d'appui entre la position de repos et la position activée, de laquelle on déduit la vitesse de déplacement par rapport au réservoir. Lorsque la distance d'activation d'un dispositif de distribution est connue, la mesure de la durée de déplacement seule permet de déduire la vitesse de déplacement.
- L'étape de mesure de la distance, et de préférence de la durée, de déplacement de la zone d'appui comprend une étape d'émission et une étape de réception d'un signal optique entre la zone d'appui et le réservoir, de préférence comprenant une étape de réflexion du signal optique sur le réservoir ou la zone d'appui. Dans un exemple, le signal optique émis de la zone d'appui (respectivement du réservoir) vers le réservoir (respectivement la zone d'appui) se réfléchit sur le réservoir (respectivement la zone d'appui) pour être reçu au niveau de la zone d'appui (respectivement le réservoir). Dans ce cas, la distance parcourue par le signal optique et sa durée de parcours, par exemple un rayon infrarouge, peuvent être environ le double respectivement de la distance et de la durée de parcours entre la zone d'appui et le réservoir. Dans un autre exemple, le signal optique émis de la zone d'appui (respectivement du réservoir) est reçu directement au niveau du réservoir (respectivement de la zone d'appui). Dans ce cas, la distance parcourue par le signal optique et la durée de parcours traduisent la distance parcourue entre la zone d'appui et le réservoir. Dans ces deux cas, une variation de la distance et de la durée de parcours du signal optique traduit une variation de la distance (ou le double de la distance) entre la zone d'appui et le réservoir et permet donc d'effectuer la mesure du déplacement de la zone d'appui par rapport au réservoir dans le temps.
- Lors de la qualification de la dose distribuée, on considère que la dose distribuée est complète si la distance de déplacement de la zone d'appui est supérieure à un seuil prédéfini, la distance de déplacement étant de préférence parcourue en une durée inférieure à un seuil prédéfini. Alternativement, on peut considérer que la dose distribuée est complète si la distance de déplacement est parcourue à une vitesse supérieure à un seuil prédéfini. Pour certains dispositifs de distribution, la distribution d'une dose complète est effectuée par le déplacement de la zone d'appui sur une distance fixe. Dans ce cas, le seuil prédéfini correspond à cette distance fixe et le terme « supérieure » est à comprendre par « supérieure ou égale ».
- L'étape de quantification d'une valeur physique comprend une étape de mesure de la pression exercée sur la zone d'appui.
- On considère que la dose distribuée est complète si l'intensité de la pression exercée dépasse un seuil prédéfini, de préférence pendant une durée supérieure à un seuil prédéfini. En effet, pour distribuer une dose complète, il est souvent préférable que l'utilisateur exerce une pression suffisante, de préférence pendant une durée suffisante, sur la zone d'appui.
- L'étape de quantification d'une valeur physique comprend une étape de mesure de l'accélération de la zone d'appui. Pour éviter les faux positifs, notamment pour distinguer le cas dans lequel tout le dispositif de distribution se déplace de celui dans lequel seule la zone d'appui se déplace par rapport au réservoir, il est intéressant de combiner la mesure de l'accélération de la zone d'appui avec la mesure de l'intensité et/ou la durée de la pression exercée sur la zone d'appui.
- L'étape de quantification d'une valeur physique comprend une étape de mesure de l'accélération de la zone d'appui et simultanément de l'accélération du réservoir, et une étape de détermination de l'accélération relative de la zone d'appui par rapport au réservoir. Grâce à la mesure des deux accélérations, d'une part on obtient l'accélération relative de la zone d'appui par rapport au réservoir par une soustraction des deux accélérations, et d'autre part on évite les faux positifs en prenant compte uniquement les déplacements de la zone d'appui par rapport au réservoir.
- Lors de la qualification de la dose distribuée, on considère que la dose distribuée est complète si l'accélération relative mesurée est supérieure à un seuil prédéfini, puis de préférence inférieure à un seuil prédéfini. On obtient généralement la distribution d'une dose complète lorsque l'utilisateur exerce une accélération suffisante sur la zone d'appui par rapport au réservoir, mais de préférence une accélération suffisamment faible pour minimiser les risques de mauvais fonctionnement.
- On considère que la quantité de produit délivré correspond à une dose complète si l'intégrale de l'accélération pendant une durée prédéfinie comprend la succession d'une valeur positive et d'une valeur négative, comprises chacune dans un intervalle de temps prédéfini. Autrement dit, la courbe de la mesure de l'accélération en fonction du temps comprend un ensemble de valeurs positives suivi d'un ensemble de valeurs négatives, ce qui correspond à une vitesse qui augmente puis diminue. La courbe de la mesure de l'accélération se décompose ainsi en trois zones.
- L'étape de quantification d'une valeur physique comprend une étape de mesure du poids du dispositif de distribution solidarisé au dispositif d'assistance.
- Au cours de l'étape de qualification de la dose distribuée, on quantifie la dose de produit distribuée. En particulier, on quantifie la dose de produit distribuée en utilisant les informations sur la distance et/ou la durée de déplacement de la zone d'appui par rapport au réservoir, et/ou l'accélération relative entre la zone d'appui et le réservoir, et/ou le poids du dispositif de distribution solidarisé au dispositif d'assistance.
- On détecte une variation brusque de l'accélération et on considère, lors de la qualification de la dose distribuée, que la dose distribuée est complète si ces variations sont d'une intensité supérieure à un seuil prédéterminé. La variation brusque de l'accélération se traduit par une variation de la dérivée seconde de la courbe de l'accélération en fonction du temps.
- On commence à enregistrer des informations sur l'intensité de la pression exercée par l'utilisateur et/ou sur l'accélération de la zone d'appui si l'intensité de la pression exercée dépasse un seuil prédéfini.

L'invention a enfin pour objet un kit de distribution d'un produit, comprenant un dispositif de distribution du produit et un dispositif d'assistance tel que décrit précédemment. De préférence le dispositif d'assistance et le dispositif de distribution sont des dispositifs distincts, rapportés l'un sur l'autre et amovibles, mais on peut envisager qu'ils soient d'un seul bloc, venus de matière.

Nous allons maintenant présenter des modes de réalisation particuliers de l'invention donnés à titre d'exemple non limitatif et à l'appui des figures annexées sur lesquelles :
- la figure 1 est une vue en perspective d'un dispositif d'assistance selon un mode de réalisation solidarisé à un dispositif de distribution,
- la figure 2 est un ensemble de deux vues du dispositif d'assistance de la figure 1, à gauche une vue en perspective explosée et à droite une vue en perspective et partiellement en transparence,
- la figure 3 est une vue en perspective d'un dispositif d'assistance selon un autre mode de réalisation solidarisé à un dispositif de distribution,
- la figure 4 est un ensemble de deux vues du dispositif d'assistance de la figure 3, à gauche une vue en perspective explosée et à droite une vue en coupe longitudinale partielle incluant un réservoir du dispositif de distribution,
- la figure 5 est un graphe montrant les étapes d'un procédé de surveillance selon deux différents modes de réalisation,
- la figure 6 est un graphique théorique montrant des valeurs physiques quantifiées et traitées par le système de traitement,
- les figures 7 à 9 sont des graphiques expérimentaux montrant des valeurs physiques quantifiées et traitées par le système de traitement.

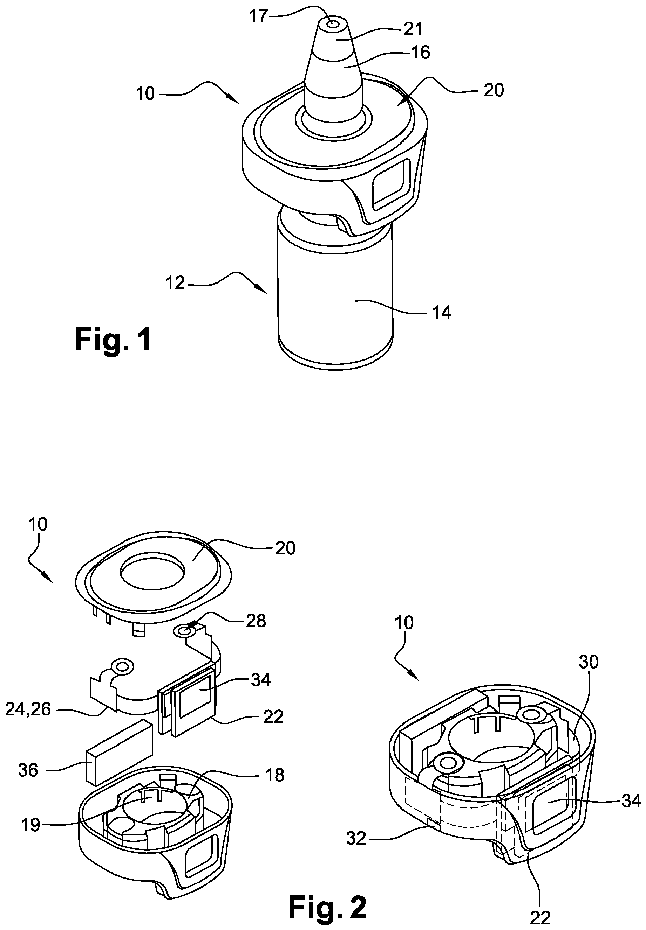

La figure 1 illustre un dispositif d'assistance 10 à l'utilisation d'un dispositif de distribution 12 à activation axiale d'un produit. Le dispositif de distribution 12 comprend un réservoir 14 et un embout de distribution 16 muni d'une buse portant un orifice de distribution 17. L'embout de distribution 16 est rapporté sur la partie mobile d'une pompe qui comprend une partie fixe, montée fixe sur le réservoir 14. Aussi, l'embout de distribution 16 comprend une zone de préhension disposée de part et d'autre et à la base de la buse portant l'orifice de distribution 17, permettant d'activer une distribution de produit. Le dispositif d'assistance 10 est rapporté sur le dispositif de distribution 12. Dans ce mode de réalisation, il est rapporté sur l'embout de distribution 16. Comme l'on peut le voir de manière plus précise sur la figure 2, le dispositif d'assistance 10 se présente sous la forme d'une collerette annulaire creuse comportant une paroi intérieure formant un col 18 dans lequel vient s'insérer l'embout de distribution 16, et une paroi extérieure concentrique, formant une enveloppe externe du dispositif d'assistance 10. Le col 18 comprend des moyens de solidarisation 19 au dispositif de distribution 12, dans cet exemple des moyens d'encliquetage du dispositif d'assistance 10sur l'embout de distribution 16. De façon alternative, voire combinée, les moyens de solidarisation 19 au dispositif de distribution 12 comprennent des moyens d'encliquetage du dispositif d'assistance 10 sur le réservoir 14.

La collerette comporte en outre une paroi supérieure supportant une zone d'appui 20. La zone d'appui 20 est montée mobile par rapport au réservoir 14 entre une position de repos et une position activée dans laquelle elle active le dispositif de distribution 12 pour obtenir la distribution de produit lorsqu'un utilisateur exerce une pression sur la zone d'appui 20 vers le réservoir 14 suivant l'axe de distribution. L'axe de distribution correspond à l'axe du réservoir, à savoir un axe vertical sur la figure 1. La zone d'appui 20 comporte deux surfaces d'appui disposées de part et d'autre de l'orifice de distribution 17 sur lesquelles l'utilisateur pose le doigt index et le doigt majeur pour exercer la force d'activation axiale.

Dans une variante parmi d'autres, l'embout de distribution, pourvu de l'orifice de distribution, pourrait faire partie du dispositif d'assistance, lequel est rapporté sur le réservoir du dispositif de distribution au moment de la solidarisation des deux dispositifs d'assistance 10 et de distribution.

Le dispositif d'assistance 10 comprend des moyens 24, 28 pour quantifier une valeur physique liée à la sollicitation exercée par l'utilisateur sur la zone d'appui 20 entre la position de repos et la position activée, cette sollicitation permettant de déplacer la zone d'appui 20. Il comporte en outre un système de traitement des informations 22 pour traiter la valeur physique quantifiée et fournir une information sur la dose de produit distribuée. Le système de traitement 22 est un système comprenant un ensemble de composants (mécaniques, électroniques, chimiques, photoniques et/ou biologiques) capable de traiter automatiquement des informations. Il comprend par exemple un circuit imprimé de type PCB (pour « printed circuit board »), un ensemble de transistors et/ou un calculateur. Le système de traitement 22 est configuré pour fournir une information quantitative sur la dose de produit distribuée, par exemple un volume de dose, une taille de goutte ou encore une répartition spatiale de goutte.

Les moyens pour quantifier une valeur physique comprennent des moyens pour mesurer la distance de déplacement 24 de la zone d'appui 20 entre la position de repos et la position activée. Les moyens pour mesurer la distance de déplacement 24 comprennent dans cet exemple des moyens optiques, en particulier un émetteur et un récepteur d'un signal optique, par exemple un rayon infrarouge. L'émetteur et le récepteur peuvent être tous les deux disposés sur une paroi de la zone d'appui 20 faisant face au réservoir 14. Lorsque l'émetteur émet un signal optique, ce dernier se réfléchit sur le réservoir 14 pour se diriger à nouveau vers la zone d'appui 20. Il est alors capté par le récepteur. On déduit la distance de déplacement de la zone d'appui en mesurant la durée entre l'émission et la réception du signal optique, la vitesse du signal optique étant connue. Alternativement, les moyens pour mesurer la distance de déplacement peuvent mesurer la distance de déplacement du réservoir (ou de tout élément fixe par rapport au réservoir) par rapport à la zone d'appui.

Les moyens pour quantifier une valeur physique comprennent en outre des moyens pour mesurer la durée de déplacement 26 de la zone d'appui 20. La mesure de la durée de déplacement peut également être assurée par les moyens optiques 24 décrits ci-dessus. L'information mesurée par les moyens pour mesurer la durée de déplacement 26 est ensuite fournie au système de traitement 22 qui en déduisent une information sur la vitesse de déplacement de la zone d'appui 20 par rapport au réservoir 14, qui peut correspondre à la vitesse de déplacement de la zone d'appui 20 par rapport à tout élément fixe par rapport au réservoir 14, en la combinant avec l'information fournie par les moyens pour mesurer la distance de déplacement, ici les moyens optiques 24. Alternativement, les moyens pour mesurer la durée de déplacement peuvent mesurer la durée de déplacement du réservoir (ou de tout élément fixe par rapport au réservoir) par rapport à la zone d'appui.

Les moyens pour quantifier une valeur physique comprennent par ailleurs des moyens pour mesurer la pression 28 exercée sur la zone d'appui 20, pour fournir une information sur l'intensité et/ou la durée de la pression exercée par l'utilisateur sur la zone d'appui 20. Les moyens pour mesurer la pression 28 comprennent ici deux capteurs de pression (« Force Sensing Resistor » ou FSR en anglais) montés sur un repli du col 18 faisant face à la zone d'appui 20. Chacun des capteurs de pression 28 est placé à l'extrémité d'une languette reliée au système de traitement 22. Un exemple de capteur de pression convenable est le capteur de pression de la marque Interlink Electronics agissant dans la gamme de forces 20 gf - 2,0 kgf (« gf » pour gramme-force, 1 gf = 9,80665 mN). Avantageusement, les moyens pour mesurer la pression 28 peuvent également servir de moyens pour activer et/ou réveiller les éventuels autres moyens pour quantifier une valeur physique lorsqu'ils détectent une pression supérieure à un seuil prédéterminé. Alternativement, les moyens pour mesurer la pression peuvent mesurer la pression subie par le réservoir (ou de tout élément fixe par rapport au réservoir) lors de l'activation.

Les moyens pour quantifier une valeur physique comprennent également des moyens pour mesurer l'accélération 30 (visibles sur la figure 2B) de la zone d'appui 20 du dispositif d'assistance 10. Les moyens pour mesurer l'accélération 30 comprennent un accéléromètre monté fixe par rapport à la zone d'appui 20. La combinaison des informations fournies par les moyens pour mesurer la pression 28 et les moyens pour mesurer l'accélération 30 permet de déterminer si la dose distribuée est complète, tout en évitant les faux positifs. Alternativement, les moyens pour mesurer l'accélération peuvent mesurer l'accélération du réservoir (ou de tout élément fixe par rapport au réservoir) lors de l'activation.

Le dispositif d'assistance 10 comprend des moyens pour mesurer le poids 32 du dispositif de distribution 12 solidarisé au dispositif d'assistance 10, configurés pour fournir une information sur la quantité de produit dans le réservoir 14. Les moyens pour mesurer le poids 32 comprennent un ou plusieurs capteurs de pression (« Force Sensing Resistor » ou FSR en anglais) disposés au-dessus du réservoir 14, de préférence de manière à pouvoir mesurer le poids du dispositif de distribution 12 quelle que soit son inclinaison.

Le système de traitement 22 comprend des moyens de lecture d'informations portées par le dispositif de distribution 12. Le dispositif de distribution 12 comprend un support d'informations lisible par voie électronique. Ce support est par exemple un QR code (ou code matriciel), ou une radio-étiquette (de type tag RFID ou « radio frequency identification » en anglais) collée sur le réservoir 14 ou l'embout de distribution 16. La radio-étiquette comprend des informations telles que, dans cet exemple, le volume de remplissage du réservoir 14, la géométrie de l'embout de distribution 16, la viscosité du produit, la date d'expiration/fabrication. Les moyens de lecture comprennent dans cet exemple une antenne apte à lire la radio-étiquette pour en extraire les informations utiles au traitement des informations.

Dans une variante, le système de traitement 22 est connecté à un objet extérieur au dispositif d'assistance 10, par exemple à un serveur, un récepteur, un réseau intranet ou l'internet.

Le système de traitement 22 comprend des moyens de stockage d'une valeur variable sur la quantité de produit restant dans le réservoir 14, par exemple une mémoire intégrée dans le système de traitement 22. Le dispositif d'assistance 10 comporte par ailleurs des moyens d'indication 34 des informations fournies par le système de traitement 22, comprenant ici un écran d'affichage 34 d'informations sous forme alphanumérique. Le dispositif d'assistance 10 comporte une batterie portable 36 pour alimenter les différents composants, en particulier les moyens pour quantifier une valeur physique et le système de traitement 22. Dans une variante, il est alimenté en énergie par une source externe. Enfin, le dispositif d'assistance 10 comporte également un capteur de température au voisinage du système de traitement 22, afin de détecter toute surchauffe dans le dispositif d'assistance.

Le dispositif d'assistance peut également comprendre des moyens de détection d'inclinaison couplés au système de traitement et aptes à détecter une mauvaise inclinaison du dispositif de distribution par l'intermédiaire de la détection de l'inclinaison du dispositif d'assistance. Le système de traitement peut être paramétré pour envoyer un signal de mauvaise utilisation à l'utilisateur ou lui indiquer la bonne façon d'utiliser le dispositif de distribution.

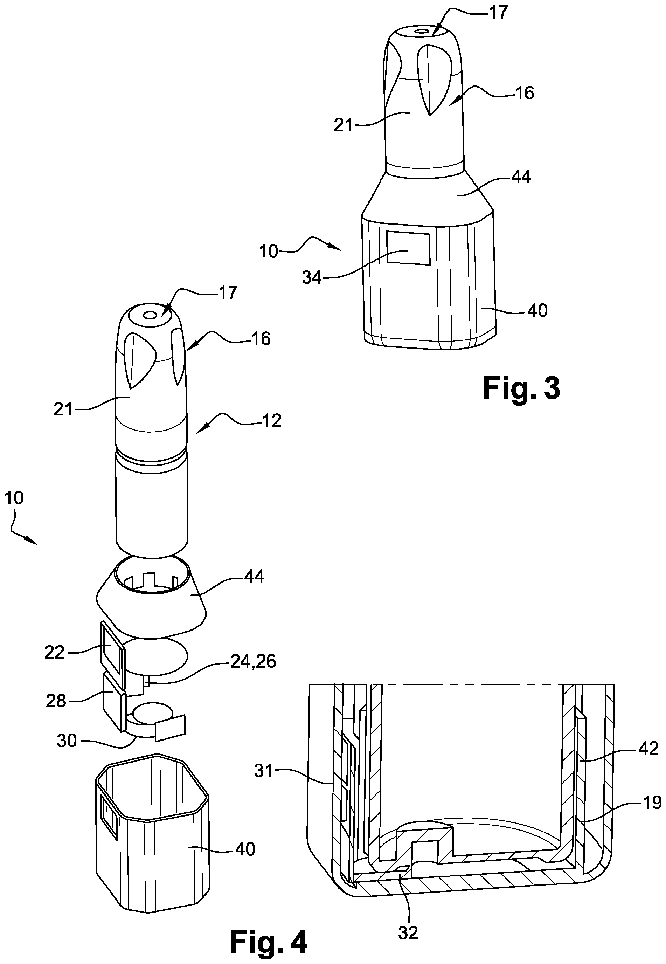

Les figures 3 et 4, illustrent un dispositif d'assistance 10 selon un autre mode de réalisation. Les mêmes références numériques sont utilisées pour les éléments en commun avec le mode de réalisation précédent.

Le dispositif d'assistance 10 comprend ici un corps principal 40, ayant un siège intérieur 42 destiné à recevoir le réservoir 14 du dispositif de distribution 12. Le siège intérieur 42 comprend des moyens de solidarisation 19 au réservoir 14. Le dispositif d'assistance 10 comprend en outre une collerette 44 ayant une ouverture par laquelle peut s'insérer le dispositif de distribution 12. L'embout de distribution 16 est similaire à celui de la figure 1. Dans le présent mode de réalisation, l'embout de distribution 16 est pourvu d'appui doigts (non représentés) faisant office de zone d'appui montée mobile par rapport au réservoir 14, lorsque l'utilisateur exerce une pression sur la zone d'appui, entre une position de repos et une position activée de distribution de produit. L'embout de distribution 16 est ici protégé par un capuchon 21.

Le dispositif d'assistance 10 comprend des moyens pour mesurer le poids 32 du dispositif de distribution 12 solidarisé au dispositif d'assistance 10, comportant un capteur de pression disposé sur le siège intérieur 42, sur lequel vient reposer le réservoir 14.

De façon similaire au premier mode de réalisation, le dispositif d'assistance 10 comprend également des moyens pour quantifier une valeur physique tels que des moyens pour mesurer la distance 24 et/ la durée 26 de déplacement de la zone d'appui 20, des moyens pour mesurer la pression 28 exercée sur la zone d'appui, des moyens pour mesurer l'accélération 30 de la zone d'appui, en particulier des moyens pour mesurer l'accélération relative 30,31 entre la zone d'appui 20 et les moyens de solidarisation 19 ou le réservoir 14, qui peuvent comporter deux accéléromètres, un premier accéléromètre 30 étant monté fixe par rapport à la zone d'appui 20 et un deuxième accéléromètre 31 étant monté fixe par rapport aux moyens de solidarisation 19, eux-mêmes montés fixes par rapport au réservoir 14. Selon une variante, les moyens pour mesurer la pression pourraient être remplacés par des moyens pour mesurer la pression disposés de façon solidaire du réservoir, par exemple par les moyens pour mesurer le poids ou encore par d'autres moyens similaires aux moyens pour mesurer la pression et configurés pour être disposés en-dessous du réservoir, au voisinage des moyens pour mesurer le poids. De tels moyens pour mesurer la pression sont des moyens pour quantifier une valeur physique liée à la sollicitation exercée par l'utilisateur sur la zone d'appui, pour fournir une information sur l'intensité et/ou la durée de la pression exercée par l'utilisateur sur la zone d'appui. Avantageusement, les moyens pour mesurer la pression peuvent également servir de moyens pour activer et/ou réveiller les éventuels autres moyens pour quantifier une valeur physique lorsqu'ils détectent une pression supérieure à un seuil prédéterminé.

Le dispositif d'assistance 10 comprend un système de traitement 22, un écran d'affichage 34 et d'autres composants similaires à celui représenté aux figures 1 et 2.

Le dispositif d'assistance 10 selon les deux modes de réalisation peut être utilisé pour surveiller la distribution d'une dose complète lors d'une activation du dispositif de distribution 12.

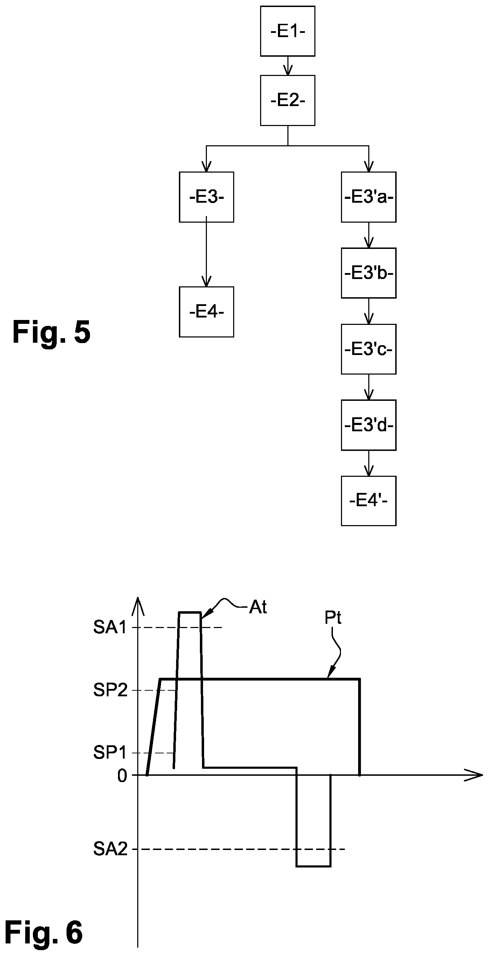

La figure 5 illustre les étapes d'un exemple de procédé de surveillance selon un premier mode de réalisation dans la branche de gauche et selon un second mode de réalisation dans la branche de droite, utilisant le dispositif d'assistance 10 des figures précédentes.

Dans une première étape E1, les moyens pour mesurer la pression 28, en tant que moyens pour activer et/ou réveiller les autres moyens, surveillent la zone d'appui 20 pour détecter une pression exercée sur celle-ci. Lorsque les moyens pour mesurer la pression 28 détectent une pression supérieure à un premier seuil de pression prédéterminé SP1 (visible à la figure 6), par exemple un seuil choisi entre 2 N et 5 N, ils activent et/ou réveillent les moyens pour mesurer l'accélération 30 et déclenchent l'enregistrement des mesures de la pression et de l'accélération en fonction du temps dans une deuxième étape E2. Dans le mode de réalisation représenté dans la branche de gauche du graphe, l'enregistrement des mesures de la pression et de l'accélération est effectué pendant une durée prédéfinie, par exemple 0,5 seconde, avant d'être arrêté dans une étape E3. Dans l'étape suivante E4, les moyens pour mesurer la pression 28 et les moyens pour mesurer l'accélération 30 envoient les mesures au système de traitement 22 qui analyse le profil des mesures reçues pour fournir une information sur la dose distribuée, par exemple en le comparant au profil théorique d'une distribution correcte de la figure 6, en particulier pour savoir si la dose distribuée a été ou non complète.

Dans le mode de réalisation représenté dans la partie droite du graphe, le traitement des informations se fait en même temps que la mesure de la pression et de l'accélération. En effet, dans une étape E3'a, les moyens pour mesurer la pression 28 surveillent la pression jusqu'à ce qu'elle dépasse un deuxième seuil de pression prédéterminé SP2 (visible à la figure 6), par exemple un seuil choisi entre 7N et 10 N. Ensuite, les moyens pour mesurer l'accélération 30 surveillent l'accélération de la zone d'appui 20 pour observer, dans le temps et dans cet ordre, une accélération supérieure à un premier seuil d'accélération prédéterminé SA1, par exemple 2g, au cours d'une étape E3'b, puis une accélération sensiblement nulle au cours d'une étape E3'c, et enfin une accélération inférieure à un deuxième seuil d'accélération prédéterminé SA2, par exemple -2g, au cours d'une étape E3'd. Si le test dans au moins une des étapes E3'a à E3'd est négatif, alors le système de traitement 22 en déduit que la dose distribuée n'est pas complète. A l'issue de l'étape E3'd, au cours d'une étape E4', les mesures de la pression et de l'accélération sont arrêtées et le système de traitement 22 évalue la durée de l'activation, *i.e.* la durée des étapes E3'a à E3'd, voire la durée de chacune des étapes E3'a à E3'd. Si la durée totale ou de chacune des étapes E3'a à E3'd est comprise dans un intervalle de durée prédéterminé, alors le système de traitement 22 en déduit que la dose distribuée est complète.

Dans les deux modes de réalisation du procédé de surveillance, le système de traitement 22 peut par exemple informer l'utilisateur sur la nature de la dose distribuée, réévaluer le nombre de doses restantes dans le réservoir 14 et/ou stocker l'information. Il peut également traiter les mesures de la pression et de l'accélération, de préférence en combinaison avec d'autres informations telles que la mesure du poids ou la mesure de la distance et/ou de la vitesse de déplacement de la zone d'appui 20, ou encore la mesure de l'accélération relative entre la zone d'appui 20 et le réservoir 14 (ou les moyens de solidarisation 19 au réservoir 14), pour fournir des informations quantitatives sur la dose distribuée.

La figure 6 est un graphique théorique représentant les mesures de la pression et de l'accélération de la zone d'appui 20 dans le temps dans le cas de la distribution d'une dose complète.

La courbe *Pt* représente l'intensité de la pression exercée sur la zone d'appui 20 en fonction du temps. Celle-ci augmente très rapidement au début de l'activation, dépassant d'abord le premier seuil de pression prédéterminé SP1, par exemple choisi entre 2 N et 5 N, à partir duquel les composants sont activés et/ou réveillés, puis le deuxième seuil de pression prédéterminé SP2, par exemple choisi entre 7 N et 10 N, à partir duquel la distribution de produit est déclenchée. L'intensité de la pression est maintenue au-dessus de ce seuil de pression prédéterminé SP2 pendant une durée supérieure à un seuil de durée prédéterminé, par exemple 0,5 seconde.

La courbe *At* représente l'accélération de la zone d'appui 20 en fonction du temps. Celle-ci a un profil en créneau et comprend trois parties distinctes. Dans une première partie, après que l'intensité de la pression a dépassé le deuxième seuil de pression prédéterminé SP2, l'accélération de la zone d'appui 20 est positive et augmente jusqu'à dépasser le premier seuil d'accélération prédéterminée SA1, par exemple 2 g. Dans une deuxième partie, l'accélération décroît jusqu'à devenir sensiblement nulle pendant une période de temps prédéterminé, par exemple 0,2 s. Puis dans une troisième partie, l'accélération devient négative et décroît jusqu'à dépasser le deuxième seuil d'accélération prédéterminée SA2, par exemple - 2 g. L'accélération croît ensuite pour s'annuler. On note que l'intégrale de l'accélération pendant une durée prédéfinie comprend la succession d'une valeur positive (première partie de la courbe *At*) et d'une valeur négative (troisième partie de la courbe *At*), comprises chacune dans un intervalle de temps prédéfini.

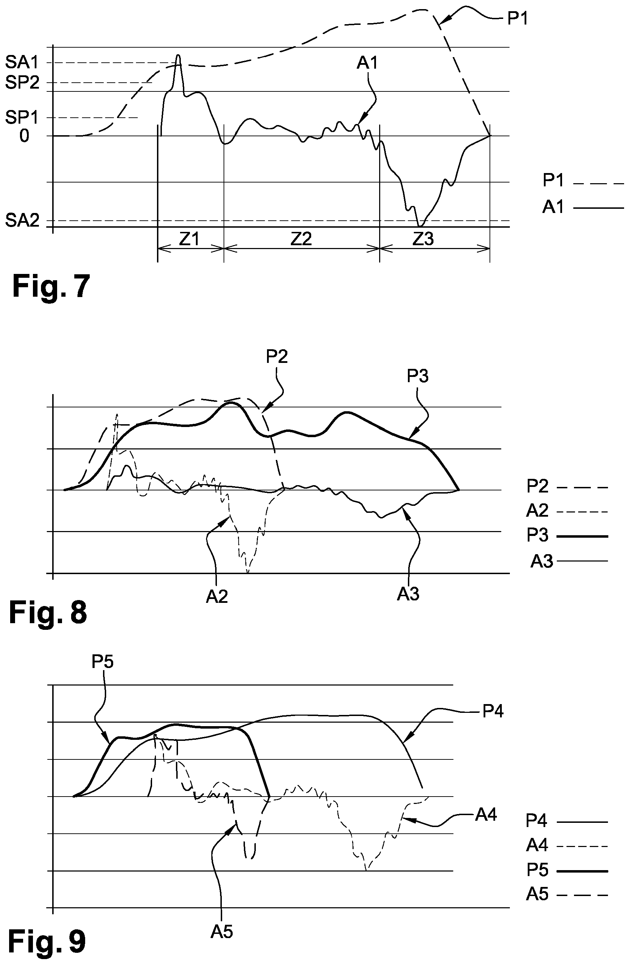

Les figures 7 à 9 sont trois graphiques expérimentaux montrant les mesures de la pression exercée sur la zone d'appui 20 et de l'accélération de la zone d'appui 20 en fonction du temps.

Le graphique de la figure 7 correspond au profil d'une courbe de pression *P1* et d'accélération *A1* correspondant à la distribution d'une dose complète. Sur la courbe de pression *P1,* on observe bien le profil d'une pression qui croît en dépassant les deux seuils de pression SP1 et SP2, puis qui est maintenue au dessus du seuil SP2 pendant une durée supérieure à une durée prédéterminée avant de décroître pour s'annuler. Sur la courbe *A1,* on distingue effectivement les trois zones distinctes de la courbe théorique *At* de la figure 6. Dans la première zone Z1 l'accélération est positive, augmente puis diminue, l'intégrale de l'accélération est positive, la vitesse de déplacement augmente. Cette première zone Z1 correspond au début de l'activation du dispositif de distribution 12. Dans la deuxième zone Z2, l'accélération est sensiblement nulle, l'intégrale de l'accélération est sensiblement nulle, la vitesse de déplacement est constante. Cette deuxième zone Z2 correspond au milieu de l'activation du dispositif de distribution 12. Dans la troisième zone Z3 l'accélération est négative, diminue puis augmente, l'intégrale de l'accélération est négative, la vitesse de déplacement diminue. Cette troisième zone Z3 correspond à la fin de l'activation du dispositif de distribution 12.

Le graphique de la figure 8 correspond au profil de deux paires de courbes de pression et d'accélération (*P2*, *A2*) et (*P3*, *A3*), la première paire correspondant à la distribution d'une dose complète et la deuxième paire correspondant à la distribution d'une dose incomplète en raison d'une durée d'activation supérieure à une durée prédéterminée ou complète mais avec un mauvais spray lorsqu'il s'agit d'une pulvérisation (mauvaise distribution des tailles de goutte ou mauvais profil de spray), par exemple 1s, à cause, par exemple, d'une pression trop faible sur ladite durée d'activation.

On constate que le profil de la paire de courbes (*P2*, *A2*) dans le temps correspond sensiblement à celui des deux courbes théoriques de la figure 6. En revanche, la courbe d'accélération *A3* ne présente pas les trois parties distinctes de la courbe *At* de la figure 6, en particulier elle ne dépasse pas dans un premier temps le premier seuil d'accélération prédéterminé SA1 et ne dépasse pas dans un deuxième temps le deuxième seuil d'accélération prédéterminé SA2. L'absence des deux pics d'accélération est notamment due à une activation avec une pression trop faible pendant une durée trop longue, donc une vitesse d'activation trop longue.

Le graphique de la figure 9 correspond au profil de deux paires de courbes de pression et d'accélération (*P4*, *A4*) et (*P5*, *A5*), la première paire correspondant à la distribution d'une dose complète et la deuxième paire correspondant à la distribution d'une dose incomplète en raison d'une durée d'activation inférieure à une durée prédéterminée, par exemple 0,5s, à cause, par exemple, d'une pression forte mais sur une durée trop faible.

On remarque que le profil de la paire de courbes (*P4*, *A4*) dans le temps correspond sensiblement à celui des deux courbes théoriques de la figure 6. En revanche, le profil de la paire de courbes (*P5*, *A5*) ne correspond pas à celui des deux courbes théoriques de la figure 6. En effet, la courbe d'accélération *A5* présente les trois parties distinctes sur une durée inférieure à la durée prédéterminée. Un tel profil d'accélération traduit un appui de l'utilisateur pendant une durée insuffisante pour déplacer la zone d'appui 20 de la position de repos à la position activée.

L'invention n'est pas limitée aux modes de réalisation présentés et d'autres modes de réalisation apparaîtront clairement à l'homme du métier. Par exemple, les moyens pour mesurer la distance et/ou la durée de déplacement peuvent comprendre d'autres moyens tels qu'un capteur à effet hall. Ce dernier peut être monté fixe par rapport à la zone d'appui 20 ou au réservoir 14 et détecte la position de l'un ou de l'autre par la présence d'une pièce mécanique ou d'un aimant par un principe connu de soi.

Par ailleurs, le dispositif de distribution 12 décrit ici est un dispositif de distribution nasale, configuré pour distribuer le produit sous forme de spray dans le nez. Il serait envisageable d'adapter le dispositif d'assistance 10 pour un autre type de dispositif de distribution.

## Revendications

1. Dispositif d'assistance (10) à l'utilisation d'un dispositif de distribution (12) à activation axiale d'un produit contenu dans un réservoir (14), la distribution étant activée par l'utilisateur suite à une pression exercée par l'utilisateur sur une zone d'appui (20) portée par le dispositif d'assistance (10) et/ou le dispositif de distribution (12), la zone d'appui (20) étant montée mobile par rapport au réservoir (14) entre une position de repos et une position activée de distribution de produit, le dispositif d'assistance (10) comprenant :
- des moyens de solidarisation (19) au dispositif de distribution (12),
- des moyens pour quantifier une valeur physique liée à la sollicitation exercée par l'utilisateur sur la zone d'appui (20) entre la position de repos et la position activée,
- un système de traitement des informations (22) pour traiter la valeur physique quantifiée et fournir une information sur la dose de produit distribuée,
**caractérisé en ce que** les moyens pour quantifier une valeur physique comprennent :
- des moyens pour mesurer la pression (28) exercée sur la zone d'appui (20), pour fournir une information sur l'intensité et/ou la durée de la pression exercée par l'utilisateur sur la zone d'appui (20), et
- des moyens pour mesurer l'accélération (30) de la zone d'appui (20).

2. Dispositif d'assistance (10) selon la revendication précédente, dans lequel les moyens pour quantifier une valeur physique comprennent des moyens pour mesurer la distance de déplacement (24) de la zone d'appui (20) entre la position de repos et la position activée, des moyens pour mesurer la durée de déplacement (26) de la zone d'appui (20), le système de traitement (22) étant apte à fournir une information sur la vitesse de déplacement de la zone d'appui (20) par rapport au réservoir (14), et dans lequel les moyens pour mesurer la distance de déplacement (24) de la zone d'appui (20) et la durée de déplacement (26) de la zone d'appui (20) comprennent des moyens optiques configurés pour mesurer la distance de déplacement et la durée de déplacement, par l'émission et la réception d'un signal optique entre la zone d'appui (20) et le réservoir (14).

3. Dispositif d'assistance (10) selon l'une quelconques des revendications précédentes, dans lequel les moyens pour quantifier une valeur physique comprennent des moyens pour mesurer l'accélération (30) relative entre la zone d'appui et le réservoir (14), de préférence :
deux accéléromètres, un premier accéléromètre étant monté fixe par rapport à la zone d'appui (20) et un deuxième accéléromètre étant monté fixe par rapport au réservoir (14), le système de traitement (22) étant apte à traiter les informations d'accélération des deux accéléromètres pour en déduire l'accélération relative entre la zone d'appui (20) et le réservoir (14).

4. Dispositif d'assistance (10) selon l'une quelconque des revendications précédentes, comprenant des moyens pour mesurer le poids (32) du dispositif de distribution (12) solidarisé au dispositif d'assistance (10), configurés pour fournir une information sur la quantité de produit dans le réservoir (14).

5. Dispositif d'assistance (10) selon l'une quelconque des revendications 2 à 4, dans lequel le système de traitement (22) est configuré pour fournir une information quantitative sur la dose de produit distribuée, par exemple un volume de dose, une taille de goutte ou encore une répartition spatiale d'un spray ou de gouttes.

6. Dispositif d'assistance (10) selon l'une quelconque des revendications précédentes, dans lequel le système de traitement (22) comprend des moyens de lecture d'informations portées par le dispositif de distribution (12).

7. Dispositif d'assistance (10) selon l'une quelconque des revendications précédentes, dans lequel le système de traitement (22) comprend des moyens de stockage d'une valeur variable sur la quantité de produit restant dans le réservoir (14).

8. Dispositif d'assistance (10) selon l'une quelconque des revendications précédentes, comprenant des moyens d'indication (34) des informations fournies par le système de traitement (22), par exemple des moyens visuels, des moyens sonores et/ou des moyens tactiles.

9. Procédé de surveillance de la distribution d'une dose complète lors d'une activation d'un dispositif de distribution de produit (12), au moyen d'un dispositif d'assistance (10) selon l'une quelconque des revendications précédentes, comprenant les étapes suivantes :
- quantification d'une valeur physique liée à la sollicitation exercée par l'utilisateur sur la zone d'appui (20) entre la position de repos et la position activée,
- qualification de la dose distribuée à partir de la valeur physique quantifiée,
dans lequel l'étape de quantification d'une valeur physique comprend une étape de mesure de la pression exercée sur la zone d'appui (20) et une étape de mesure de l'accélération de la zone d'appui (20).

10. Procédé de surveillance selon la revendication précédente, dans lequel l'étape de quantification d'une valeur physique comprend une étape de mesure de la distance de déplacement de la zone d'appui entre la position de repos et la position activée, et une étape de mesure de la durée de déplacement de la zone d'appui (20) entre la position de repos et la position activée, de laquelle on déduit la vitesse de déplacement par rapport au réservoir (14), et procédé dans lequel l'étape de mesure de la distance de déplacement de la zone d'appui, et l'étape de mesure de la durée de déplacement de la zone d'appui (20) comprennent une étape d'émission et une étape de réception d'un signal optique entre la zone d'appui (20) et le réservoir (14), de préférence comprenant une étape de réflexion du signal optique sur le réservoir (14) ou la zone d'appui (20).

11. Procédé de surveillance selon l'une quelconque des revendications 9 à 10, au cours duquel, lors de la qualification de la dose distribuée, on considère que la dose distribuée est complète si la distance de déplacement de la zone d'appui (20) est supérieure à un seuil prédéfini, la distance de déplacement étant de préférence parcourue en une durée inférieure à un seuil prédéfini ; ou si l'intensité de la pression exercée dépasse un seuil prédéfini (SP2), de préférence pendant une durée supérieure à un seuil prédéfini.

12. Procédé de surveillance selon l'une quelconque des revendications 9 à 11, dans lequel l'étape de quantification d'une valeur physique comprend une étape de mesure de l'accélération de la zone d'appui (20) et simultanément de l'accélération du réservoir (14), et une étape de détermination de l'accélération relative de la zone d'appui (20) par rapport au réservoir (14).

13. Procédé de surveillance selon la revendication précédente, au cours duquel, lors de la qualification de la dose distribuée, on considère que la dose distribuée est complète si l'accélération relative mesurée est supérieure à un seuil prédéfini (SA1) puis de préférence inférieure à un seuil prédéfini (SA2).

14. Procédé de surveillance selon l'une quelconque des revendications 9 à 13, au cours duquel on considère que la quantité de produit délivré correspond à une dose complète si l'intégrale de l'accélération pendant une durée prédéfinie comprend la succession d'une valeur positive et d'une valeur négative, comprises chacune dans un intervalle de temps prédéfini.

15. Procédé de surveillance selon l'une quelconque des revendications 9 à 14, dans lequel l'étape de quantification d'une valeur physique comprend une étape de mesure du poids du dispositif de distribution (12) solidarisé au dispositif d'assistance (10).

## Patentansprüche

1. Hilfsvorrichtung (10) zur Verwendung einer Abgabevorrichtung (12) mit axialer Aktivierung eines in einem Behälter (14) enthaltenen Produkts, wobei die Abgabe durch den Benutzer infolge eines Drucks aktiviert wird, den der Benutzer auf einen Auflagebereich (20) ausübt, der von der Hilfsvorrichtung (10) und/oder der Abgabevorrichtung (12) getragen wird, wobei der Auflagebereich (20) in Bezug auf den Behälter (14) zwischen einer Ruhestellung und einer aktivierten Stellung zur Abgabe des Produkts beweglich angebracht ist, wobei die Hilfsvorrichtung (10) aufweist:
- Mittel zur festen Verbindung (19) mit der Abgabevorrichtung (12),
- Mittel zum Quantifizieren eines physikalischen Werts, der mit der Beanspruchung verbunden ist, die der Benutzer zwischen der Ruhestellung und der aktivierten Stellung auf den Auflagebereich (20) ausübt,
- ein Informationsverarbeitungssystem (22), um den quantifizierten physikalischen Wert zu verarbeiten und eine Information über die abgegebene Produktdosis zu liefern,
**dadurch gekennzeichnet, dass** die Mittel zum Quantifizieren eines physikalischen Wertes aufweisen:
- Mittel zum Messen des Drucks (28), der auf den Auflagebereich (20) ausgeübt wird, um eine Information über die Intensität und/oder die Dauer des Drucks zu liefern, den der Benutzer auf den Auflagebereich (20) ausübt, und
- Mittel zum Messen der Beschleunigung (30) des Auflagebereichs (20).

2. Hilfsvorrichtung (10) nach dem vorhergehenden Anspruch, wobei die Mittel zum Quantifizieren eines physikalischen Wertes Mittel zum Messen der Bewegungsstrecke (24) des Auflagebereichs (20) zwischen der Ruheposition und der aktivierten Position aufweisen, Mittel zum Messen der Bewegungsdauer (26) des Auflagebereichs (20) aufweisen, wobei das Verarbeitungssystem (22) dazu ausgelegt ist, eine Information über die Bewegungsgeschwindigkeit des Auflagebereichs (20) in Bezug auf den Behälter (14) zu liefem, und wobei die Mittel zum Messen der Bewegungsstrecke (24) des Auflagebereichs (20) und der Bewegungsdauer (26) des Auflagebereichs (20) optische Mittel aufweisen, die so konfiguriert sind, dass sie die Bewegungsstrecke und die Bewegungsdauer messen, indem sie ein optisches Signal zwischen dem Auflagebereich (20) und dem Behälter (14) aussenden und empfangen.

3. Hilfsvorrichtung (10) nach einem der vorhergehenden Ansprüche, wobei die Mittel zum Quantifizieren eines physikalischen Wertes Mittel zum Messen der relativen Beschleunigung (20) zwischen der Auflagezone und dem Behälter (14) aufweisen, vorzugsweise:
zwei Beschleunigungsmesser, wobei ein erster Beschleunigungsmesser in Bezug auf den Auflagebereich (20) fest montiert ist und ein zweiter Beschleunigungsmesser in Bezug auf den Behälter (14) fest montiert ist, wobei das Verarbeitungssystem (22) ausgestaltet ist, die Beschleunigungsinformationen der beiden Beschleunigungsmesser zu verarbeiten, um daraus die relative Beschleunigung zwischen dem Auflagebereich (20) und dem Behälter (14) abzuleiten.

4. Hilfsvorrichtung (10) nach einem der vorhergehenden Ansprüche, aufweisend Mittel zum Messen des Gewichts (32) der fest mit der Hilfsvorrichtung (10) verbundenen Abgabevorrichtung (12), die so konfiguriert sind, dass sie eine Information über die Menge des Produkts im Behälter (14) liefern.

5. Hilfsvorrichtung (10) nach einem der Ansprüche 2 bis 4, wobei das Verarbeitungssystem (22) so konfiguriert ist, dass es eine quantitative Information über die abgegebene Produktdosis liefert, beispielsweise ein Dosisvolumen, eine Tropfengröße oder auch eine räumliche Verteilung eines Sprays oder von Tropfen.

6. Hilfsvorrichtung (10) nach einem der vorhergehenden Ansprüche, wobei das Verarbeitungssystem (22) Mittel zum Lesen von Informationen aufweist, die von der Abgabevorrichtung (12) getragen werden.

7. Hilfsvorrichtung (10) nach einem der vorhergehenden Ansprüche, wobei das Verarbeitungssystem (22) Mittel zur Speicherung eines variablen Wertes über die Menge des im Behälter (14) verbleibenden Produkts aufweist.

8. Hilfsvorrichtung (10) nach einem der vorhergehenden Ansprüche, die Mittel zur Anzeige (34) der vom Verarbeitungssystem (22) gelieferten Informationen aufweist, z. B. visuelle Mittel, akustische Mittel und/oder taktile Mittel.

9. Verfahren zur Überwachung der Abgabe einer vollen Dosis bei Aktivierung einer Produktabgabevorrichtung (12) mittels einer Hilfsvorrichtung (10) nach einem der vorhergehenden Ansprüche, mit den folgenden Schritten:
- Quantifizieren eines physikalischen Werts, der mit der Beanspruchung zusammenhängt, die der Benutzer zwischen der Ruheposition und der aktivierten Position auf den Auflagebereich (20) ausübt,
- Qualifizieren der abgegebenen Dosis anhand des quantifizierten physikalischen Werts, wobei der Schritt des Quantifizierens eines physikalischen Werts einen Schritt des Messens des auf den Auflagebereich (20) ausgeübten Drucks und einen Schritt des Messens der Beschleunigung des Auflagebereichs (20) aufweist.

10. Überwachungsverfahren nach dem vorhergehenden Anspruch, wobei der Schritt des Quantifizierens eines physikalischen Werts einen Schritt des Messens der Bewegungsstrecke des Auflagebereichs zwischen der Ruheposition und der aktivierten Position, und einen Schritt des Messens der Bewegungsdauer des Auflagebereichs (20) zwischen der Ruheposition und der aktivierten Position aufweist, aus dem die Bewegungsgeschwindigkeit in Bezug auf den Behälter (14) abgeleitet wird, und Verfahren, bei dem der Schritt des Messens der Bewegungsstrecke der Auflagezone und der Schritt des Messens der Bewegungsdauer des Auflagebereichs (20) einen Schritt des Sendens und einen Schritt des Empfangens eines optischen Signals zwischen dem Auflagebereich (20) und dem Behälter (14) aufweisen, vorzugsweise einen Schritt der Reflexion des optischen Signals an dem Behälter (14) oder dem Auflagebereich (20) aufweisen.

11. Überwachungsverfahren nach einem der Ansprüche 9 bis 10, bei dem beim Qualifizieren der abgegebenen Dosis davon ausgegangen wird, dass die abgegebene Dosis vollständig ist, wenn die Bewegungsstrecke des Auflagebereichs (20) größer als ein vordefinierter Schwellenwert ist, wobei die Bewegungsstrecke vorzugsweise in einer Zeitdauer zurückgelegt wird, die kleiner als ein vordefinierter Schwellenwert ist; oder wenn die Intensität des ausgeübten Drucks einen vordefinierten Schwellenwert (SP2) überschreitet, vorzugsweise für eine Zeitdauer, die größer als ein vordefinierter Schwellenwert ist.

12. Überwachungsverfahren nach einem der Ansprüche 9 bis 11, wobei der Schritt des Quantifizierens einer physikalischen Größe einen Schritt des Messens der Beschleunigung des Auflagebereichs (20) und gleichzeitig der Beschleunigung des Behälters (14), sowie einen Schritt des Bestimmens der relativen Beschleunigung des Auflagebereichs (20) in Bezug auf den Behälter (14) aufweist.

13. Überwachungsverfahren nach dem vorhergehenden Anspruch, bei dem bei der Qualifizierung der abgegebenen Dosis davon ausgegangen wird, dass die abgegebene Dosis vollständig ist, wenn die gemessene relative Beschleunigung größer als ein vordefinierter Schwellenwert (SA1) und dann vorzugsweise kleiner als ein vordefinierter Schwellenwert (SA2) ist.

14. Überwachungsverfahren nach einem der Ansprüche 9 bis 13, bei dem davon ausgegangen wird, dass die abgegebene Produktmenge einer vollständigen Dosis entspricht, wenn das Integral der Beschleunigung über eine vordefinierte Zeitdauer die Aufeinanderfolge eines positiven und eines negativen Wertes aufweist, die jeweils in einem vordefinierten Zeitintervall liegen.

15. Überwachungsverfahren nach einem der Ansprüche 9 bis 14, wobei der Schritt des Quantifizierens eines physikalischen Werts einen Schritt des Messens des Gewichts der Abgabevorrichtung (12), die fest mit der Hilfsvorrichtung (10) verbunden ist, aufweist.

## Claims

1. Assistance device (10) for assisting in the use of an axially activated dispensing device (12) for dispensing a product contained in a tank (14), the dispensing being activated by the user following a pressure exerted by the user on a bearing area (20) carried by the assistance device (1) and/or the dispensing device (12), the bearing area (20) being mounted so as to be able to move with respect to the tank (14) between a rest position and an activated product dispensing position, the assistance device (10) comprising:
- means for connection (19) to the dispensing device (12),
- means for quantifying a physical value linked to the force exerted by the user on the bearing area (20) between the rest position and the activated position,
- an information processing system (22) for processing the quantified physical value and providing information on the dispensed product dose,
**characterized in that** the means for quantifying a physical value comprise:
- means for measuring the pressure (28) exerted on the bearing area (20), in order to provide information on the magnitude and/or the duration of the pressure exerted by the user on the bearing area (20) and
- means for measuring the acceleration (30) of the bearing area (20).

2. Assistance device (10) according to the preceding claim, wherein the means for quantifying a physical value comprise means for measuring the distance of displacement (24) of the bearing area (20) between the rest position and the activated position, means for measuring the duration of displacement (26) of the bearing area (20), the processing system (22) being capable of providing information on the speed of displacement of the bearing area (20) with respect to the tank (14), and wherein the means for measuring the distance of displacement (24) and the duration of displacement (26) of the bearing area (20) comprise optical means configured to measure the distance of displacement and the duration of displacement, by the transmission and reception of an optical signal between the bearing area (20) and the tank (14).

3. Assistance device (10) according to any one of the preceding claims, wherein the means for quantifying a physical value comprise means for measuring the relative acceleration (30) between the bearing area and the tank (14), preferably: two accelerometers, a first accelerometer being fixedly mounted with respect to the bearing area (20) and a second accelerometer being fixedly mounted with respect to the tank (14), the processing system (22) being capable of processing the acceleration information provided by the two accelerometers in order to deduce the relative acceleration between the bearing area (20) and the tank (14).

4. Assistance device (10) according to any one of the preceding claims, comprising means for measuring the weight (32) of the dispensing device (12) connected to the assistance device (10), configured to provide information on the amount of product in the tank (14).

5. Assistance device (10) according to any one of claims 2 to 4, wherein the processing system (22) is configured to provide quantitative information on the dispensed product dose, for example a dose volume, a drop size, or a spatial distribution of a spray or drops.

6. Assistance device (10) according to any one of the preceding claims, wherein the processing system (22) comprises means for reading information carried by the dispensing device (12).

7. Assistance device (10) according to any one of the preceding claims, wherein the processing system (22) comprises means for storing a variable value corresponding to the amount of product remaining in the tank (14).

8. Assistance device (10) according to any one of the preceding claims, comprising means for indicating (34) the information provided by the processing system (22), for example visual means, audible means and/or tactile means.

9. Method for monitoring the dispensing of a complete dose during the activation of a product dispensing device (12), by means of an assistance device (10) according to any one of the preceding claims, comprising the following steps:
- quantifying a physical value linked to the force exerted by the user on the bearing area (20) between the rest position and the activated position,
- qualifying the dispensed dose using the quantified physical value,
wherein the step of quantifying a physical value comprises a step of measuring the pressure exerted on the bearing area (20) and a step of measuring the acceleration of the bearing area (20).

10. Monitoring method according to the preceding claim, wherein the step of quantifying a physical value comprises a step of measuring the distance of displacement of the bearing area between the rest position and the activated position, and a step of measuring the duration of displacement of the bearing area (20) between the rest position and the activated position, which can be used to deduce the speed of displacement with respect to the tank (14), and monitoring method wherein the step of measuring the distance of displacement of the bearing area (20), and the step of measuring the duration of displacement of the bearing area (20) comprise a step of transmitting and a step of receiving an optical signal between the bearing area (20) and the tank (14), preferably comprising a step of reflecting the optical signal on the tank (14) or on the bearing area (20).

11. Monitoring method according to any one of claims 9 to 10, during which, when qualifying the dispensed dose, the dispensed dose is considered to be complete if the distance of displacement of the bearing area (20) is greater than a predefined threshold, the distance of displacement preferably being travelled in a duration less than a predefined threshold; or if the magnitude of the pressure exerted exceeds a predefined threshold (SP2), preferably for a duration greater than a predefined threshold.

12. Monitoring method according to any one of claims 9 to 11, wherein the step of quantifying a physical value comprises a step of measuring the acceleration of the bearing area (20) and simultaneously the acceleration of the tank (14), and a step of determining the relative acceleration of the bearing area (20) with respect to the tank (14).

13. Monitoring method according to the preceding claim, during which, when qualifying the dispensed dose, the dispensed dose is considered to be complete if the relative acceleration measured is greater than a predefined threshold (SA1), then preferably less than a predefined threshold (SA2).

14. Monitoring method according to any one of claims 9 to 13, during which the amount of product dispensed is considered to correspond to a complete dose if the integral of the acceleration for a predefined duration comprises the succession of a positive value and a negative value, each within a predefined time interval.

15. Monitoring method according to any one of claims 9 to 14, wherein the step of quantifying a physical value comprises a step of measuring the weight of the dispensing device (12) connected to the assistance device (10).
